# EUROPÄISCHE PATENTSCHRIFT

(11) **EP 0 967 997 B1**
(45) Veröffentlichungstag und Bekanntmachung des Hinweises auf die Patenterteilung: **19.11.2003**
(21) Anmeldenummer: 98924027.0
(22) Anmeldetag: 18.03.1998
(51) Int. Cl.: A61K 41/00, A61K 38/13, A61K 31/475, A61K 45/06, A61K 31/275

(54) **PHARMAZEUTISCHE ZUSAMMENSETZUNG ZUR AUFHEBUNG DER MEMBRANVERMITTELTEN RESISTENZ VON ZELLEN**
PHARMACEUTICAL COMPOSITION FOR ELIMINATING MEMBRANE-MEDIATED CELL RESISTANCE
COMPOSITION PHARMACEUTIQUE DESTINEE A SUPPRIMER LA RESISTANCE CELLULAIRE D'ORIGINE MEMBRANAIRE

(30) Priorität: 19.03.1997 DE 19711503
(43) Veröffentlichungstag der Anmeldung: 05.01.2000
(73) Patentinhaber: Deutsches Krebsforschungszentrum Stiftung des öffentlichen Rechts, 69120 Heidelberg (DE)
(72) Erfinder: GRANZOW, Christof, D-69121 Heidelberg (DE); PONSTINGL, Herwig, D-69120 Heidelberg (DE); HEFFT, Irmgard, D-69126 Heidelberg (DE); KOPUN-GRANZOW, Marijana, D-69121 Heidelberg (DE); GROS, Gabriele, D-69124 Heidelberg (DE); STÖHR, Michael, D-69437 Neckargerach (DE)
(74) Vertreter: Schüssler, Andrea, Dr.
(86) Internationale Anmeldenummer: DE9800814
(87) Internationale Veröffentlichungsnummer: WO98041236

(56) Entgegenhaltungen:
- AGUERO ET AL.: "Verapamil increases rhodamine 123 laser phototherapy of drug-resistant human sarcoma cells" J. CLIN. LASER MED. SURG., Bd. 12, Nr. 4, 1994, Seiten 193-198, XP002074265
- MAZIERE ET AL.: "Potentialisation de l'effet photocytotoxique du Photofrin II; actions synergiques du vérapamil et de la lovastatine " BULL. ACAD. NATL. MED., Bd. 178, Nr. 6, 1994, Seiten 1177-1189, XP002074266
- MOTE ET AL.: "Paclitaxel sensitizes multidrug resistant cells to radiation" ANTI-CANCER DRUGS , Bd. 7, Nr. 2, 1996, Seiten 182-188, XP002074267
- CHORVATH ET AL.: " Non-immunosuppressive cyclosporine derivative PSC 833 abolishes resistance of human multidrug-resistant ovarian carcinoma cells in vitro to paclitaxel and paclitaxel-induced radiosensitization" INT. J. CANCER, Bd. 72, Nr. 5, 4.September 1997, Seiten 916-917, XP002074268
- KELLEN: "The reversal of multidrug resistance in cancer" ANTICANCER RESEARCH, Bd. 13, Nr. 4, 1993, Seiten 959-961, XP002074269

## Beschreibung

Die Erfindung betrifft eine pharmazeutische Zusammensetzung zur Aufhebung der membranvermittelten Resistenz von Zellen, insbesondere der membranvermittelten Mehrfach-Chemoresistenz von Tumorzellen, sowie ein Verfahren hierzu und die Verwendung dieser Zusammensetzung.

Aguero et al., J. Clin. Laser Med. Surg., Bd. 12, Nr. 4, S. 193-198 (1994) betrifft die Behandlung von Tumorzellen (HT1080 Fibrosarcoma-Zellen) mit Verapamil und Rhodamin 123 sowie die nachfolgende Bestrahlung mit einem Argon-Laser.

Maziere et al., Bull. Acad. Natl. Med., Bd. 178, Nr. 6, S. 1177-1189 (1994) betrifft die Potenzierung des phototoxischen Effekts von Photofrin II durch eine synergistische Wirkung von Verapamil und Lovastatin, die jeweils als Einzelsubstanzen verabreicht werden.

In Mote et al., Anti-Cancer Drugs, Bd. 7, Nr. 2, S. 182-188 (1996) wird der bekannte Synergismus von Chemo- und Strahlentherapie bei chemosensiblen Tumorzellen durch ionisierende ⁶⁰Co-Bestrahlung nach Paclitaxel-Vorbehandlung bestätigt. Bei einer "multidrug"-resistenten Variante ist hierfür zusätzlich die Einwirkung von Verapamil erforderlich.

Beim Zustandekommen der membranvermittelten Resistenz pro- und eukaryontischer Zellen, insbesondere der Cytostatikaresistenz von Tumorzellen, spielen membranständige Transportmechanismen in vielen Fällen eine Schlüsselrolle. Ein Beispiel eines solchen membranständigen Transportmechanismus beruht auf der Aktivität von P-Glykoprotein. Das P-Glykoprotein ist ein Transmembranprotein mit der Funktion eines zellulären Auswärtstransports von Substraten. Substrate dieses membranständigen Transportmechanismus sind viele klinisch bedeutsame Cytostatika. Dies bedeutet, daß die Cytostatika durch diesen membranständigen Transportmechanismus aus der Zelle transportiert (eliminiert) werden. Derartige Zellen sind somit gegen Cytostatika resistent. Inhibitoren dieses membranständigen Transportmechanismus wurden bisher bei Tumorpatienten eingesetzt, um den Auswärtstransport und somit die Resistenz zu vermindern. Derartige Inhibitoren müssen jedoch in hoher Konzentration über einen sehr langen Zeitraum, der sich oft über bis zu mehrere Tage erstreckt, verabreicht werden. Daher treten Nebenwirkungen auf, die den Organismus sehr stark belasten. Ferner wird bei der Verabreichung dieser Inhibitoren lediglich eine vorübergehende Minderung, jedoch keine Aufhebung der Resistenz erreicht.

Der vorliegenden Erfindung liegt somit die Aufgabe zugrunde, eine pharmazeutische Zusammensetzung bereitzustellen, mit der die membranvermittelte Resistenz von Zellen, insbesondere von Tumorzellen, rasch und vollständig ohne nennenswerte Nebenwirkungen aufgehoben wird.

Erfindungsgemäß wird dies durch die Gegenstände in den Patentansprüchen erreicht.

Gegenstand der vorliegenden Erfindung ist somit eine pharmazeutische Zusammensetzung, enthaltend einen Inhibitor für Membranpumpen und ein photoaffinitätsmarkiertes Chemotherapeutikum.

Der Ausdruck "Inhibitor für Membranpumpen" umfaßt Verbindungen jeglicher Art, die membranständige Transportmechanismen, insbesondere Transportmechanismen für den Auswärtstransport, z. B. einen durch P-Glykoprotein vermittelten Auswärtstransport, hemmen (blockieren). Eine Vielzahl derartiger Inhibitoren sind dem Fachmann bekannt. Besonders günstige Inhibitoren sind Verapamil und seine Derivate sowie Cyclosporin A und seine Derivate, z.B. PSC 833. Es können auch mehrere verschiedene dieser Inhibitoren in der erfindungsgemäßen pharmazeutischen Zusammensetzung vorliegen.

Der Ausdruck "photoaffinitätsmarkiertes Chemotherapeutikum" umfaßt Verbindungen jeglicher Art, die ein Chemotherapeutikum und eine Photoaffinitätsmarkierung aufweisen. Chemotherapeutika sind Verbindungen, die zur Behandlung von durch Bakterien, Viren, Protozoen, Pilzen und Würmern verursachten Infektionskrankheiten sowie von Krebserkrankungen geeignet sind, wobei Cytostatika besonders bevorzugt sind. Photoaffinitätsmarkierungen sind Verbindungen, die durch Bestrahlung mit Licht in eine Form umgewandelt werden können, die unter Ausbildung kovalenter Bindungen mit zellulären Bestandteilen reagiert. Eine Vielzahl derartiger Photoaffinitätsmarkierungen sind dem Fachmann bekannt. Als günstig hat es sich erwiesen, wenn die Photoaffinitätsmarkierung eine Azid-Gruppierung aufweist, da diese unter physiologischen Bedingungen besonders gut in eine Form umgewandelt werden kann, die an zelluläre Bestandteile bindet. Besonders bevorzugt ist das photoaffinitätsmarkierte Chemotherapeutikum Napavin. Dieses leitet sich vom Vinblastin ab, bei dem eine Acetylgruppe durch eine Azid-aufweisende Gruppe ersetzt ist. In der erfindungsgemäßen pharmazeutischen Zusammensetzung können mehrere verschiedene photoaffinitätsmarkierte Chemotherapeutika vorliegen.

In der erfindungsgemäßen Zusammensetzung beträgt das molare Verhältnis von Inhibitor : photoaktivitätsmarkiertem Chemotherapeutikum 1:10 bis 10:1, vorzugsweise 5:1 bis 3:1.

Eine erfindungsgemäße Zusammensetzung kann übliche Hilfsstoffe enthalten. Als Hilfsstoffe können die üblichen, wie Arzneimittelträger, Bindemittel, Sprengmittel, Gleitmittel, Lösungsmittel, Lösungsvermittler, Freigabe-Beschleuniger, Freigabe-Verzögerer, Emulgatoren, Stabilisatoren, Färbemittel oder Geschmackskorrigenzien verwendet werden. Die erfindungsgemäße pharmazeutische Zusammensetzung kann auch in Form einer sterilen physiologischen Kochsalzlösung vorliegen, d.h. Inhibitor und photoaffinitätsmarkiertes Chemotherapeutikum sind in einer sterilen physiologischen Kochsalzlösung gelöst. Das Lösen erfolgt ggfs. unter Zusatz eines Lösungsvermittlers, wie Alkohol, z.B. Ethanol und Benzylalkohol.

Die Menge des Inhibitors und des photoaffinitätsmarkierten Chemotherapeutikums in der pharmazeutischen Zusammensetzung kann von 0,1 bis 10, insbesondere ca. 1 mg pro 1000 g Zusammensetzung betragen.

Die Applikation der erfindungsgemäßen Zusammensetzung kann in üblicher Weise erfolgen, z.B. lokal, topisch oder systemisch. Zweckmäßig ist es, wenn die erfindungsgemäße Zusammensetzung während eines Zeitraumes von 5 bis 30 Minuten, besonders bevorzugt von ca. 10 Minuten, intraarteriell regional, d.h. zu bestimmten Bereichen des Körpers über versorgende Arterien zugeführt wird. Unmittelbar anschließend wird direkt, z.B. bei oberflächlich gelegenen Zellen, oder endoskopisch belichtet. Die Dauer der Belichtung beträgt 1 bis 5, insbesondere ca. 1 Minute. Als Lichtquelle kann dabei Laserlicht oder inkoherentes Licht, ggfs. unter Verwendung von Filtern, eingesetzt werden. Die Wellenlänge und die Intensität des Lichts wird dabei in Abhängigkeit von der eingesetzten Photoaffinitätsmarkierung gewählt.

Die Applikation kann 1 bis 5, insbesondere 1 bis 3 und ganz besonders 1 mal täglich erfolgen. Die Applikation kann an mehreren hintereinander folgenden Tagen durchgeführt werden.

Gegenstand der vorliegenden Erfindung ist auch ein Verfahren zur Aufhebung der membranvermittelten Resistenz von Zellen, umfassend die folgenden Schritte:
a) Einwirken einer erfindungsgemäßen Zusammensetzung auf Zellen und
b) Belichten der Zellen.

Bei den Zellen handelt es sich um Zellen, die eine membranvermittelte Resistenz, insbesondere eine membranvermittelte Mehrfach-Chemoresistenz, besitzen. Sie können in einer üblichen Zellkultur vorliegen. Vorzugsweise sind die Zellen Tumorzellen. Die Einwirkung der erfindungsgemäßen Zusammensetzung erfolgt über einige Minuten, vorzugsweise 5 bis 30 und besonders bevorzugt ca. 10 Minuten. Die Belichtung erfolgt wie vorstehend beschrieben. Die Belichtungsdauer beträgt wenige Minuten, vorzugsweise 1 bis 5 und besonders bevorzugt ca. 1 Minute. Nach der Belichtung können die Zellen kultiviert werden. Der Zellzuwachs wird nach der Kultivierung (z.B. nach drei Tagen) gemessen und die 50 %ige Hemmkonzentration in üblicher Weise graphisch ermittelt. Mit diesem Verfahren können die der membranvermittelten Resistenz zugrunde liegenden Mechanismen studiert werden.

Die vorliegende Erfindung zeichnet sich dadurch aus, daß durch das Zusammenwirken eines Inhibitors für Membranpumpen und eines photoaktivitätsmarkierten Chemotherapeutikums in Verbindung mit einer Belichtung die membranvermittelte Resistenz von Zellen, insbesondere Tumorzellen, aufgehoben wird. Die Aufhebung erfolgt dabei rasch. Ferner sind nur kurze Einwirkzeiten und geringe Substanzmengen bei der Applikation der erfindungsgemäßen pharmazeutischen Zusammensetzung notwendig. Somit ist die erfindungsgemäße Zusammensetzung nebenwirkungsarm. Sie wirkt nicht genotoxisch (erbgutschädigend). Die Belichtung erfolgt nur über eine kurze Zeitspanne, so daß keine unerwünschte Erwärmung der Zellen oder Gewebe stattfindet. Des weiteren wird bei Verabreichung der erfindungsgemäßen Zusammensetzung das Wachstum von resistenten Tumorzellen, selbst von solchen mit sehr hoher Ausprägung der membranvermittelten Mehrfach-Chemoresistenz, und von nicht-resistenten Tumorzellen unterbunden.

Die erfindungsgemäße Zusammensetzung ist gemeinsam mit dem erfindungsgemäßen Verfahren zur Resistenzunterdrückung bestens geeignet, Zelltodprogramme (Apoptose) auszulösen. Sie eignet sich deshalb zur Behandlung von Erkrankungen, bei denen es zum Auftreten einer membranvermittelten Resistenz, insbesondere einer membranvermittelten Mehrfach-Chemoresistenz, kommen kann, wie bei Infektionserkrankungen und Tumorerkrankungen, insbesondere Tumorerkrankungen der Haut, der urothelialen und rektalen Gewebe, der des oberen Aerodigestivtrakts einschließlich des Ösophagus und der im Bereich von Pleura und Peritoneum. Unter den Ausdruck Tumorerkrankungen fallen Primärtumore und Metastasen.

Das folgende Beispiel erläutert die Erfindung.

### Beispiel: Aufhebung P-Glykoprotein-vermittelter Mehrfach-Chemoresistenz von Mäuseasciteszellen und menschlichen Karzinomzellen.

Es wurden chemosensitive Mäuseasciteszellen (Stamm HD34K) mit ihrer durch P-Glykoproteinvermittelten Mehrfach-Chemoresistenzausgezeichneten Variante (Stamm SR) verglichen. Die Chemoresistenz der SR-Zellen ist spontan entstanden. Die Mäuseasciteszellen wurden vor Versuchsbeginn in frischem Zellkulturmedium suspendiert.

Des weiteren wurden chemosensitive KB-Zellen (menschliche Karzinomzellen) mit ihrer chemoresistenten Variante KBC5-8 verglichen. Die Mehrfach-Chemoresistenz letzterer ist gleichfalls durch P-Glykoprotein vermittelt und wurde experimentell durch Colchicinzusatz zum Zellkulturmedium induziert. Die menschlichen Karzinomzellen wurden vor Versuchsbeginn trypsiniert und in frischem Zellkulturmedium suspendiert.

Sämtliche Experimente wurden unter flavinschützenden Testbedingungen (Granzow et al., Cancer Res. 55, 4837-4843, (1995)) durchgeführt. Frisch bereitete Zellsuspensionen wurden 15 Minuten bei 36,5 °C vorinkubiert. Anschließend wurden die Zellen mit abgestuften Konzentrationen von Vinblastin und Napavin (photoaktivitätsmarkiertes Cytostatikum) inkubiert. Die Inkubation erfolgte entweder in Abwesenheit von Verapamil (Inhibitor für P-Glykoprotein) oder, im Falle von Vinblastin, mit 1 µM Verapamil bzw., im Falle von Napavin, mit 1 oder 2 µM Verapamil. Die Mäuseasciteszellen wurden mit diesen Pharmaka fünf Minuten lang inkubiert; die menschlichen Tumorzellen wurden zehn Minuten inkubiert. Dann wurden die Zellsuspensionen in Plastikschalen umpipettiert und bei Raumtemperatur 1 Minute lang mit einem Argonlaser (457,8 nm, 48 mW/cm²) belichtet. Anschließend wurden die Zellen zweimal mit frischem Kulturmedium gewaschen und in 24-Loch-Multischalen drei Tage lang kultiviert. Am Versuchsende wurde der Zellzuwachs gemessen und die 50 %ige Hemmkonzentration der Cytostatika Vinblastin und Napavin graphisch in üblicher Weise ermittelt. Die Ergebnisse sind in der folgenden Tabelle dargestellt.

**Tabelle:**

| **50 %ige mikromolare Hemmkonzentrationen** | | | | | |
|---|---|---|---|---|---|
| **Zellstamm** | **VLB** | **VLB + 1µM VPM** | **NAP** | **NAP + 1µM VPM** | **NAP + 2µM VPM** |
| KB (w.t.) | 0,16 +/-0,01 | 0,18 +/- 0,01 | 0,16 | 0,15 | n.d. |
| KBC5-8 (mdr) | 27 +/-1 | 28 | 3,8 +/-0,9 | 1,0 +/-0,1 | 0,35 +/-0,15 |
| HD34K (w.t.) | 0,66 | 0,75 | 0,43 +/-0,05 | 0,33 +/-0,01 | n.d. |
| SR (mdr) | 7 | 9 | 4,75 +/-0,5 | 0,9 +/-0,15 | 0,6 |
| Abkürzungen: w.t.: chemosensible Wildtyp-Zellen; mdr: mehrfachchemoresistente Varianten; n.d.: nicht bestimmt; VLB: Vinblastin; VPM: Verapamil; NAP: Napavin. | | | | | |

Wie aus der Tabelle zu entnehmen ist, lag bei den chemosensiblen menschlichen KB-Zellen die zur 50 %igen Wachstumsunterdrückung benötigten Konzentrationen von Vinblastin und Napavin zwischen 0.1 und 0.2 µM. Verapamil hatte darauf keinen Einfluß. Bei der mehrfach resistenten Variante KBC5-8 wurde für den gleichen Effekt die ca. 150fache Vinblastinkonzentration benötigt. Das entspricht einem exzessiven Resistenzgrad. Verapamil hatte darauf keinen Einfluß. Mit Napavin war ohne Verapamil das 23fache der bei KB-Zellen benötigten Konzentration erforderlich; mit 1 µM Verapamil zusätzlich im Ansatz war es noch das 6fache, mit 2 µM Verapamil lediglich das doppelte. In Anbetracht der Variationsbreiten besteht kein signifikanter Unterschied mehr zum Wildtyp, d.h. die Resistenz wurde aufgehoben.

Bei den chemosensiblen HD34K-Asciteszellen der Maus lag die 50 %ige Hemmkonzentration von Vinblastin bei 0.66 µM ohne Verapamil, in Gegenwart von 1 µM Verapamil unbedeutend darüber. Bei Napavin-Zusatz wurden für denselben Effekt ca. 0.4 µM benötigt, bei Zusatz von 1 µM Verapamil geringfügig weniger. Bei der mehrfachresistenten Variante SR trat 50 %ige Wachstumshemmung mit Vinblastin beim ca. 1 Ofachen der Wildtypkonzentration ein. Das entspricht einem mittleren Resistenzniveau, wie es bei menschlichen, mehrfachresistenten Tumoren beobachtet wird. Mit Napavin ist dieses Verhältnis in Abwesenheit von Verapamil gleichfalls 10 zu 1. Mit 1 µM Verapamil im Ansatz hingegen sind die gemessenen Hemmkonzentrationen nicht mehr signifikant verschieden, mit 2 µM Verapamil werden sie identisch.

Zusammenfassend ist festzustellen, daß mehrfachchemoresistente Zellen bei der gemeinsamen Verabreichung von Napavin und Verapamil und anschließender Belichtung wieder ebenso chemosensibel wie ihre parentalen Wildtypzellen werden, d.h. eine vollständige Aufhebung der. Resistenz ist zu beobachten.

## Patentansprüche

1. Pharmazeutische Zusammensetzung, enthaltend einen Inhibitor für Membranpumpen und ein photoaffinitätsmarkiertes Chemotherapeutikum.

2. Pharmazeutische Zusammensetzung nach Anspruch 1, **dadurch gekennzeichnet, daß** der Inhibitor für Membranpumpen ein Inhibitor für Membranpumpen für den Auswärtstransport ist.

3. Pharmazeutische Zusammensetzung nach Anspruch 2, **dadurch gekennzeichnet, daß** der Inhibitor ein Inhibitor des durch P-Glykoprotein-vermittelten Transports ist.

4. Pharmazeutische Zusammensetzung nach Anspruch 3, **dadurch gekennzeichnet, daß** der Inhibitor Verapamil, Cyclosporin A oder ein Derivat von diesen ist.

5. Pharmazeutische Zusammensetzung nach einem der Ansprüche 1 bis 4, **dadurch gekennzeichnet, daß** das Chemotherapeutikum ein Cytostatikum ist.

6. Pharmazeutische Zusammensetzung nach einem der Ansprüche 1 bis 5, **dadurch gekennzeichnet, daß** die Photoaffinitätsmarkierung ein Azid aufweist.

7. Pharmazeutische Zusammensetzung nach einem der Ansprüche 1 bis 6, **dadurch gekennzeichnet, daß** das photoaffinitätsmarkierte Chemotherapeutikum Napavin ist.

8. In-vitro-Verfahren zur Aufhebung membranvermittelter Resistenz von Zellen, umfassend die folgenden Schritte:
a) Einwirken einer pharmazeutischen Zusammensetzung nach einem der Ansprüche 1 bis 7 auf Zellen und
b) Belichten der Zellen.

9. Verfahren nach Anspruch 8, **dadurch gekennzeichnet, daß** das Belichten mit Laser oder koherentem Licht erfolgt.

10. Verfahren nach Anspruch 8 oder 9, **dadurch gekennzeichnet, daß** die Zellen Tumorzellen sind.

11. Verwendung eines Inhibitors für Membranpumpen und eines photoaffinitätsmarkierten Chemotherapeutikums zur Herstellung einer pharmazeutischen Zusammensetzung zur Aufhebung der membranvermittelten Resistenz von Zellen.

12. Verwendung nach Anspruch 11 zur Auslösung von Apoptose in Tumorzellen.

## Claims

1. Pharmaceutical composition comprising an inhibitor for membrane pumps and a photoaffinity labelled chemotherapeutic agent.

2. Pharmaceutical composition according to claim 1, **characterised in that** the inhibitor for membrane pumps is an inhibitor for membrane pumps for the outward transport.

3. Pharmaceutical composition according to claim 2, **characterised in that** the inhibitor is an inhibitor of the P-glycoprotein-mediated transport.

4. Pharmaceutical composition according to claim 3, **characterised in that** the inhibitor is verapamil, cyclosporin A or a derivative of these.

5. Pharmaceutical composition according to anyone of claims 1 to 4, **characterised in that** the chemotherapeutic agent is a cytostatic agent.

6. Pharmaceutical composition according to anyone of claims 1 to 5, **characterised in that** the photoaffinity label includes an azide.

7. Pharmaceutical composition according to anyone of claims 1 to 6, **characterised in that** the photoaffinity labelled chemotherapeutic agent is napavin.

8. In-vitro method for removing membrane-mediated resistance from cells comprising the steps of:
a) Applying a pharmaceutical composition according to anyone of claims 1 to 7 on cells and
b) Exposing the cells.

9. Method according to claim 8, **characterised in that** the exposure takes place by laser or by coherent light.

10. Method according to claim 8 or 9, **characterised in that** the cells are tumour cells.

11. Use of an inhibitor for membrane pumps and a photoaffinity labelled chemotherapeutic agent for the preparation of a pharmaceutical composition for removing of the membrane-mediated resistance of cells.

12. Use according to claim 11 for inducing apoptosis in tumour cells.

## Revendications

1. Composition pharmaceutique, contenant un inhibiteur pour pompes membranaires et un agent chimiothérapeutique à marquage de photoaffinité.

2. Composition pharmaceutique suivant la revendication 1, **caractérisée en ce que** l'inhibiteur pour pompes membranaires est un inhibiteur pour pompes membranaires pour le transport vers l'extérieur.

3. Composition pharmaceutique suivant la revendication 2, **caractérisée en ce que** l'inhibiteur est un inhibiteur du transport effectué sous la médiation de P-glycoprotéines.

4. Composition pharmaceutique suivant la revendication 3, **caractérisée en ce que** l'inhibiteur est le vérapamil, la cyclosporine A ou un de leurs dérivés.

5. Composition pharmaceutique suivant l'une des revendications 1 à 4, **caractérisée en ce que** l'agent chimiothérapeutique est un cytostatique.

6. Composition pharmaceutique suivant l'une des revendications 1 à 5, **caractérisée en ce que** le marquage de photoaffinité comporte un azide.

7. Composition pharmaceutique suivant l'une des revendications 1 à 6, **caractérisée en ce que** l'agent chimiothérapeutique à marquage de photoaffinité est la napavine.

8. Procédé in vitro de suppression de la résistance sous médiation membranaire de cellules, comprenant les étapes suivantes :
a) action sur les cellules d'une composition pharmaceutique suivant l'une des revendications 1 à 7, et
b) exposition des cellules à la lumière.

9. Procédé suivant la revendication 8, **caractérisé en ce que** l'exposition est effectuée par laser ou avec de la lumière cohérente.

10. Procédé suivant la revendication 8 ou 9, **caractérisé en ce que** les cellules sont des cellules tumorales.

11. Utilisation d'un inhibiteur pour pompes membranaires et d'un agent chimiothérapeutique à marquage de photoaffinité pour la préparation d'une composition pharmaceutique destinée à supprimer la résistance sous médiation membranaire de cellules.

12. Utilisation suivant la revendication 11 pour provoquer l'apoptose dans des cellules tumorales.
